# EUROPEAN PATENT APPLICATION

(11) **EP 4 756 023 A1**
(43) Date of publication of application: **10.06.2026**
(21) Application number: 24217937.2
(22) Date of filing: 06.12.2024
(51) Int. Cl.: C12N 15/113, C12N 15/67

(54) **SELF-CLEAVABLE RIBOZYME THERAPEUTIC NUCLEIC ACID MOLECULES**

(71) Applicant: Max-Planck-Gesellschaft zur Förderung der Wissenschaften e.V., 80539 München (DE)
(72) Inventor: The designation of the inventor has not yet been filed
(74) Representative: Vossius & Partner Patentanwälte Rechtsanwälte mbB

(57) **Abstract**

The present invention relates to a therapeutic nucleic acid molecule comprising or consisting of an open reading frame comprising (i) a part of a gene, wherein the part comprises (a) at least one intron and the flanking exons of the gene, (b) at least two exons of the gene, wherein the first exon encodes the 5'UTR or the last exon encodes the 3'UTR, (c) at least one exon of the gene preceded by an exogenous 5'UTR or followed by an exogenous 3'UTR, or (d) at least one exon of the gene, wherein the gene is associated with a disease in humans or is useful for treating a disease in humans, wherein the disease can be treated by the expression of the gene itself or one of its functional or sequence paralogs; and (ii) inserted within the part of a gene according to (i) a nucleic acid sequence encoding a self-cleavable ribozyme, wherein (a') in (a) the nucleic acid sequence encoding a self-cleavable ribozyme is inserted into the intron, (b') in (b) the nucleic acid sequence encoding a self-cleavable ribozyme is inserted into the 5' or 3'UTR, (c') in (c) the nucleic acid sequence encoding a self-cleavable ribozyme is inserted into the exogenous 5' or 3'UTR, and (d') in (d) the nucleic acid sequence encoding a self-cleavable ribozyme is inserted into at least one exon.

## Description

The present invention relates to a therapeutic nucleic acid molecule comprising or consisting of an open reading frame comprising (i) a part of a gene, wherein the part comprises (a) at least one intron and the flanking exons of the gene, (b) at least two exons of the gene, wherein the first exon encodes the 5'UTR or the last exon encodes the 3'UTR, (c) at least one exon of the gene preceded by an exogenous 5'UTR or followed by an exogenous 3'UTR, or (d) at least one exon of the gene, wherein the gene is associated with a disease in humans or is useful for treating a disease in humans, wherein the disease can be treated by the expression of the gene itself or one of its functional or sequence paralogs; and (ii) inserted within the part of a gene according to (i) a nucleic acid sequence encoding a self-cleavable ribozyme, wherein (a') in (a) the nucleic acid sequence encoding a self-cleavable ribozyme is inserted into the intron, (b') in (b) the nucleic acid sequence encoding a self-cleavable ribozyme is inserted into the 5' or 3'UTR, (c') in (c) the nucleic acid sequence encoding a self-cleavable ribozyme is inserted into the exogenous 5' or 3'UTR, and (d') in (d) the nucleic acid sequence encoding a self-cleavable ribozyme is inserted into at least one exon.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Organisms utilize several mechanisms to compensate for the damaging consequences of genetic perturbations. One such mechanism is the newly identified process of transcriptional adaptation (TA). TA is a mechanism contributing to genetic robustness (Rossi et al. (2015), Nature. 524 (7564): 230-233). It regulates gene expression in response to mRNA degradation, independently of protein feedback loops. TA-inducing approaches rely on introducing a premature termination codon to trigger mRNA degradation / mRNA decay.

TA is a relatively newly discovered form of genetic compensation. Genetic compensation is the phenomenon whereby the effect of a deleterious mutation is buffered by the genome. TA can upregulate genes that compensate for the loss of the mutant gene function, thereby masking the expected mutant phenotype (Zacharias and Stainier (2020), Trends in Genetics, 36(12):926-935). It was found that specific mutations in a given mRNA resulted in faster decay of that mRNA and induced increased transcription of genes in the same transcript family. They named these paralogs "adapting genes" (El-Brolosy et al. (2019), Nature, 568:193-197).

In transcriptional adaptation transcripts harboring premature termination codons (PTCs) undergo degradation through processes such as but not limited to nonsense-mediated decay (NMD), producing cleaved mRNA molecules (Rossi et al. (2015), Nature. 524, 230-3). These fragmented mRNA molecules and/or their derivatives are then used by the cell to target other genes or loci, termed adapting genes. In some cases, these adapting genes can act as functional paralogs to the PTC-bearing transcript (El-Brolosy et al. (2019), Nature, 568, 193-197, PMID: 31951195), thereby leading to functional compensation.

It was found herein for the first time that via TA the compensation for the loss of a mutant gene function cannot only be induced by "normal" cellular mRNA decay but that via TA said compensation can also be induced through the expression of a "minigene" containing a self-cleaving ribozyme.

Accordingly, the present invention relates in first aspect to a therapeutic nucleic acid molecule comprising or consisting of an open reading frame comprising (i) a part of a gene, wherein the part comprises (a) at least one intron and the flanking exons of the gene, (b) at least two exons of the gene, wherein the first exon encodes the 5'UTR or the last exon encodes the 3'UTR, (c) at least one exon of the gene preceded by an exogenous 5'UTR or followed by an exogenous 3'UTR, or (d) at least one exon of the gene, wherein the gene is associated with a disease in humans or is useful for treating a disease in humans, wherein the disease can be treated by the expression of the gene itself or one of its functional or sequence paralogs; and (ii) inserted within the part of a gene according to (i) a nucleic acid sequence encoding a self-cleavable ribozyme, wherein (a') in (a) the nucleic acid sequence encoding a self-cleavable ribozyme is inserted into the intron, (b') in (b) the nucleic acid sequence encoding a self-cleavable ribozyme is inserted into the 5' or 3'UTR, (c') in (c) the nucleic acid sequence encoding a self-cleavable ribozyme is inserted into the exogenous 5' or 3'UTR, and (d') in (d) the nucleic acid sequence encoding a self-cleavable ribozyme is inserted into at least one exon.

The term "nucleic acid molecule" in accordance with the present invention includes DNA, such as cDNA or double or single stranded genomic DNA and RNA. In this regard, "DNA" (deoxyribonucleic acid) means any chain or sequence of the chemical building blocks adenine (A), guanine (G), cytosine (C) and thymine (T), called nucleotide bases, that are linked together on a deoxyribose sugar backbone. DNA can have one strand of nucleotide bases, or two complimentary strands which may form a double helix structure. "RNA" (ribonucleic acid) means any chain or sequence of the chemical building blocks adenine (A), guanine (G), cytosine (C) and uracil (U), called nucleotide bases, that are linked together on a ribose sugar backbone. RNA typically has one strand of nucleotide bases, such as mRNA. Included are also single- and double-stranded hybrids molecules, i.e., DNA-DNA, DNA-RNA and RNA-RNA. The nucleic acid molecule may also be modified by many means known in the art. Non-limiting examples of such modifications include methylation, "caps", substitution of one or more of the naturally occurring nucleotides with an analog, and internucleotide modifications such as, for example, those with uncharged linkages (e.g., methyl phosphonates, phosphotriesters, phosphoroamidates, carbamates, etc.) and with charged linkages (e.g., phosphorothioates, phosphorodithioates, etc.). Nucleic acid molecules, in the following also referred as polynucleotides, may contain one or more additional covalently linked moieties, such as, for example, proteins (e.g., nucleases, toxins, antibodies, signal peptides, poly-L-lysine, etc.), intercalators (e.g., acridine, psoralen, etc.), chelators (e.g., metals, radioactive metals, iron, oxidative metals, etc.), and alkylators. The polynucleotides may be derivatized by formation of a methyl or ethyl phosphotriester or an alkyl phosphoramidate linkage. Further included are nucleic acid mimicking molecules known in the art such as synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA), peptide nucleic acid (PNA) and locked nucleic acid (LNA) (see Braasch and Corey, Chem Biol 2001, 8: 1). LNA is an RNA derivative in which the ribose ring is constrained by a methylene linkage between the 2'-oxygen and the 4'-carbon. Also included are nucleic acids containing modified bases, for example thio-uracil, thio-guanine and fluoro-uracil. A nucleic acid molecule typically carries genetic information, including the information used by cellular machinery to make proteins and/or polypeptides. The nucleic acid molecule of the invention may additionally comprise promoters, enhancers, response elements, signal sequences, polyadenylation sequences, introns, 5'- and 3'- noncoding regions, and the like.

The term "therapeutic nucleic acid molecule" designates a nucleic acid molecule that when being administered to a subject can exert a therapeutically beneficial effect; e.g. the treatment or prevention of a disease in a subject. In the context of the first aspect of the invention the term "therapeutic" is optional and may also be omitted. The therapeutic nucleic acid molecule of the invention is also referred to herein as "minigene".

The term "open reading frame" refers to nucleic acid sequence of adjacent codons that starts with a start codon, followed by a series of codons for amino acids, and ends with a stop codon. This sequence has the potential to be translated into a (poly)peptide product.

The open reading frame according to the invitation only comprise a part of a gene encoding a full-length protein. This part can comprise of (a) at least one intron and the flanking exons of the gene, (b) at least two exons of the gene, wherein the first exon encodes the 5'UTR (= 5' untranslated region) or the last exon encodes the 3'UTR (= 3' untranslated region), (c) at least one exon of the gene preceded by an exogenous 5'UTR or followed by an exogenous 3'UTR, or (d) at least one exon of the gene, wherein the gene is associated with a disease in humans or is useful for treating a disease in humans, wherein the disease can be treated by the expression of the gene itself or one of its functional or sequence paralogs.

With respect to options (b) and (c) it is noted that in the case of (b) the part comprises the naturally occurring 5'UTR or 3'UTR encoding exon of the gene while in the case of (c) the part comprises the a exogenous 5'UTR or 3'UTR encoding exon. The exogenous 5'UTR and 3'UTR encoding exons are distinct from the naturally occurring 5'UTR or 3'UTR encoding exons of the gene. Exogenous UTRs can be derived from other genes that are distinct in sequence from the naturally occurring 5'UTR or 3'UTR encoding exons of the gene from which the minigene is derived, or they designed in silico and produced by nucleotide synthesis (see, for example, Eisenhut et al. (2020), Nucleic Acids Research, 48(20):e119).

Among options (a) to (d), options (a) and (b) are preferred and option (a) is most preferred. Option (a) is illustrated by the appended examples.

The at least one intron and the flanking exons of the gene is preferably one intron and the flanking exons, two introns and the flanking exons, three introns and the flanking exons, four introns and the flanking exons, five introns and the flanking exons or six introns and the flanking exons. One intron and the flanking exons is most preferred because this is illustrated by the appended examples. It is noted that the flanking exons can be one or more exons. Three exons on each side of the one intron are illustrated by the appended examples.

The gene from which the part is derived is associated with a disease in humans or is useful for treating a disease in humans. Genes being associated with a disease in humans or being useful for treating a disease in humans are preferably genes being associated with a genetic disorder. A genetic disorder is a disease being caused in whole or in part by a change in the DNA sequence away from the normal sequence. Genetic disorders can be caused by a mutation in the gene (monogenic disorder), by mutations in multiple genes (multifactorial inheritance disorder), by a combination of gene mutations and environmental factors, or by damage to chromosomes (changes in the number or structure of entire chromosomes, the structures that carry genes). Non-limiting but preferred examples of genes being associated with a disease in humans or being associated with a disease in humans.

Herein, the disease is generally one that can be treated by the expression of the gene from which the part is derived itself or one of its functional or sequence paralogs. The present invention is based on the recently identified mode of genetic compensation that is known as "transcriptional adaptation", wherein deleterious mutations leading to mutant mRNA degradation trigger the upregulation of so-called adapting genes. In some cases the adapting gene can be the same as the mutated gene, thereby resulting in functional compensation of the mutated gene. In some cases these adapting gene can be a functional and structural paralogs of the mutated gene, thereby also resulting in functional compensation of the mutated gene. The disease is preferably one that can be treated by the expression of one of its functional or sequence paralogs, as is illustrated in the appended examples based on Duchenne muscular dystrophy (DMD) as the mutated gene and utrophin (UTRN) as the functional or sequence paralog.

Paralogs are generally homologous genes that have diverged from each other as a consequence of gene duplication. For instance, Gene 1 in the ancestral species undergoes a duplication event generating Gene 1a and Gene 1b. The ancestral species splits into two species, each with its own copy of Gene 1a and Gene 1b. Gene 1a and Gene 1b are paralogs.

In the case of a functional paralog Gene 1a and Gene 1b may be functionally redundant; i.e. in general the genes encode functionally redundant protein, wherein Protein 1a can at least in part substitute for the function of Protein 1b (and *vice versa*)*.* In the case of a sequence paralog Gene 1a and Gene 1b and the encoded Protein 1a and Protein 1b may also be sequences that share a high degree of sequence identity, preferably at least 70%, more preferably at least 80% and mots preferably at least 90% on the nucleotide and/or protein level. The sequence identity herein is preferably determined by nucleotide and/or protein BLAST. A paralog may also be at the same time, both a functional and a sequence paralog. A functional paralog is preferred.

The part of a gene according to (i) of the first aspect of the invention in addition has an insertion of a nucleic acid sequence encoding a self-cleavable ribozyme, wherein (a') in (a) the nucleic acid sequence encoding a self-cleavable ribozyme is inserted into the intron, (b') in (b) the nucleic acid sequence encoding a self-cleavable ribozyme is inserted into the 5' or 3'UTR, (c') in (c) the nucleic acid sequence encoding a self-cleavable ribozyme is inserted into the exogenous 5' or 3'UTR, and (d') in (d) the nucleic acid sequence encoding a self-cleavable ribozyme is inserted into at least one exon.

A self-cleavable ribozyme catalyzes the sequence-specific intramolecular cleavage of RNA and can be engineered to catalyze cleavage of appropriate substrates in an intermolecular fashion, thus acting as a true catalyst (for review Peng et al. (2021), RSC Chem. Biol., 2021, 2:1370-1383). Non-limiting but preferred examples of self-cleavable ribozymes will be provided herein below.

In accordance with a preferred embodiment, the therapeutic nucleic acid molecule is formulated as a lipid particle.

In accordance with a more preferred embodiment, the lipid particle is composed of ionizable lipids, helper phospholipids, cholesterol, and/or polyethylene glycol-lipids.

Lipid particles have attracted great interest because of their specificity and the duration of action they offer from the standpoint of drug delivery. Lipid particle have been used to effectively deliver therapeutic oligonucleotides, such as siRNA and ASOs *in vivo* into cells (Zimmermann et al. (2006) Nature, 441:111-114). The lipid particles are preferably composed of ionizable lipids, helper phospholipids, cholesterol, and/or polyethylene glycol-lipids (Albertsen et al. (2022), Adv Drug Deliv Rev.; 188: 114416).

In accordance with a further preferred embodiment the therapeutic nucleic acid molecule is delivered with one or more nanocarriers selected from liposomes, peptides, dendrimers, exosomes, spherical nucleic acid (SNAs), or DNA nanostructures. In accordance with another preferred embodiment the therapeutic nucleic acid molecule is used as naked therapeutic nucleic acid molecule.

It is shown in the appended examples that a self-cleaving ribozyme inserted into a Duchenne muscular dystrophy (DMD) wild-type (WT) minigene in wild-type cells causes utrophin (UTRN) upregulation. Utrophin upregulation has been proposed to be a compensatory mechanism to partially counteract the lack of dystrophin, making such a therapeutic nucleic acid molecules of the invention a promising therapeutic strategy for treating DMD patients. It was found that enriching the cleaved fragments in the nucleus via the self-cleaving results in a greater fold increase in UTRN upregulation to that observed with the NMD-minigenes without self-cleaving. To this end, a modified variant of the S. mansoni hammerhead ribozyme, hereafter referred to as T3H38-HHR, was inserted into intron 31 of the DMD WT minigene consisting of E30 to E34. HHRs are small self-cleaving ribozymes that fold into defined structures with fast cleavage kinetics in cis. By inserting this HHR into an intron, we can restrict the cleaved fragments to the nucleus. Importantly, a single nucleotide substitution alters T3H38-HHR from its catalytically active form (denoted as T3H38-aHHR) to an inactive form (denoted as T3H38-iHHR), which served as a control for cleavage efficiency. It was found that WT HEK293T cells, myotubes, HAP1 cells, and Hela cells transfected with the T3H38-aHHR construct showed a decrease in exogenous DMD mRNA levels as well as an increase in UTRN mRNA levels compared with WT cells transfected with the T3H38-iHHR construct.

In summary, the appended examples illustrate based on DMD/UTRN that upon the insertion of a ribozyme sequence into a minigene, which is then introduced into target cells wherein the minigene is transcribed, the ribozyme catalyzes the cleavage of the minigene and modulates the transcription of target genes through TA. This approach can activate a compensatory gene network in genetic diseases. Also what is needed for such therapeutically suitable minigene is (part of) the mRNA sequence (as this is where the active fragments will come from) (active as in being able to upregulate itself or a genetic/functional paralog), and the ribozyme to induce the cleavage of this mRNA sequence. This technology can be applied to a plethora of diseases that can be treated by the expression of the gene itself from which the part is derived or one of its functional or sequence paralogs.

While the appended examples illustrate option (a) of the first aspect of the invention it is at least highly plausible that also options (b) to (d) of the first aspect work. This is because what is needed to trigger TA is RNA fragments and these fragments can be generated by the activity of the self-cleaving ribozyme, which itself can be inserted anywhere into a minigene; i.e., into an intron, a 5' or 3'UTR, or in an exon itself.

While the appended examples illustrate that a disease that can be treated by the expression of its functional or sequence paralog, it is at least highly plausible that a disease can also be treated by the expression of a gene from which the minigene is derived *per se.* Self-TA (or the increase in transcription of the mutated gene itself) has been observed by the inventors in all cases that were examined thus far. In fact, evidence now points to a scenario whereby TA initially appeared, during evolution that is, as self-TA. So, when a genetic or environmental insult occurred that led to a reduction in mRNA levels of a gene (because of mRNA decay), a feedback mechanism evolved to upregulate its own expression (a concept akin to 'buffering'). During gene/genome duplication, the cis-regulatory elements required for self-TA went along with the paralog(s), and now the mRNA decay fragments of a gene can regulate itself as well as its paralogs. Thus, in the DMD/UTRN case, a ribozyme-containing UTRN minigene is also believed to be able to upregulate UTRN expression.

In accordance with a preferred embodiment of the first aspect of the invention (1) a mutation in the gene is associated with a disease in humans, (2) the gene has a functional or sequence paralog in the genome of humans, and (3) the disease can be treated by one of its functional or sequence paralog(s).

The roles of paralogous genes in disease and health are different. In most cases of Mendelian diseases, only one of the paralogous genes is associated with the disease. In 87% of the gene pairs, only one pair is associated with disease, and this trend is observed in gene families with more than two members. Once a gene is duplicated, purifying selection pressure on one or both of the copies is relaxed and they become more prone to accumulating mutations. This divergence can lead to sub-functionalization or neofunctionalization often resulting in different roles of paralogs in disease (Adebali et al. (2016), Genet Med; 18(10):1029-1036).

In accordance with a preferred embodiment of the first aspect of the invention the gene is *DMD, LMNA, FBN1, CFTR, RHO, MYH7, TP53, LDLR, HFE, FGFR3, HEXA, PAH, FBN2, HNRNPH1, SOD1, SPTLC1, ACTL6B, GJB2, MYO7A, TMC1, COL11A2, KCNQ4, SLC26A4* or *COCH* and is preferably *DMD.*

In accordance with a related more preferred embodiment of the first aspect of the invention the functional or sequence paralog is *UTRN, NEFL FBN2, TMEM16A, NEFL, OPN1MW*/*OPN1MW2*/*OPN1MW3*/*OPN1LW*/*OPN1SW*/*OPN3*/*OPN4*/*RRH*/*OPN5, MYH6, TP73*/*TP63, LPR8*/*VLDLR*/*LRP1*/*LRP1B*/*LRP4*/*LRP2*/*EGF*/*NID1*/*NID2*/*LRP5*/*LRP6*/*LRP12*/*LRP12*/*LRP3, HLA-FIHLA-C*/*HLA-G*/*HLA-A*/*HLAE*/*AZGP1*/*MR1*/*MICB*/*FCGRT*/*MICA*/*CD1A*/*CD1C*/*CD1D*/*CD18*/*CD1E*/*RAET1L*/ *ULBP1*/*RAET1G*/*ULBP2*/*ULBP3*/*RAET1E,FGFR2*/*FGFR1*/*FGFR4*/*RET*/*KDR*/*FLT4*/*FLT1*/*KIT*/*FLT3*/*PDGFRB*/ *PDGFRA*/*CSF1R*/*ROR2*/*TIE2*/*TEK*/*ROS1*/*MST1R*/*ROR1*/*EPHA4*/*INSR*/*IGF1R*/*EPHA2*/*ALK*/*AXL*/*NTRK2*/*EP HA5*/*EPHA7*/*EPHAS*/*MET*/*ER884*/*EPHA3*/*EPH83*/*TYR03*/*MUSK*/*EPH84*/*ER882*/*EPAH1*/*NTRK3*/*EPH82*/ *EGFR*/*EPHB1*/*EPHA6*/*MERTK*/*NRTK1*/*EPHA10*/*LTK*/*EPHB6*/*INSRR*/*ERBB3*/*DDR2*/*DDR1*/*LMTK2*/*RYK, HEXB, TPH2*/*TPH1*/*TH, FBN1, HRNPH2, CCS*/*SOD3, SPTLC21SPTLC31ALAS11GCATIALAS2, ACTL6A*/*ACTB*/*ACTG1*/*ACTC1*/*ACTA2*/*ACTG2*/*ACTA1*/*ACTBL2*/*ACTR1A*/*ACTR18*/*ACTL78*/*ACTR38*/*ACTR T2*/*ACTR3*/*ACTRT1*/*ACTL9*/*ACTRT3*/*ACTR2*/*ACTL7A*/*ACTLS*/*ACTRS*/*ACTRS*/*ACTR10*/*ACTR6*/*ACTL10*/*AC TR3C, GJB61GJB11GJB31GJA81GJB41GJA31GJA41GJB51GJA11GJB71GJA101GJA91GJA51GJD21GJC21 GJC1*/*GJD3*/*GJD4*/*GJET1, MYO7B*/*MYH6*/*MYH7*/*MYH8*/*MYH4*/*MYH1*/*MYH2*/*MYH3*/*MYH7B*/*MYH9*/ *MYH13*/*MYH10*/*MYH15*/*MY010*/*MYH14*/*MY058*/*MY05A*/*MY098*/*MY09A*/*MY015A*/*MY05C*/*MY03A* /*MYO16*/*MYO18*/*MYO18A*/*MYO188*/*MYO1E*/*MYO1C*/*MYO1A*/*MYO6*/*MYO38*/*MYO1D*/*MYO1F*/*MYO1 G*/*MYO1H*/*MYO19*/*CCDC158*/*CGNL1*/*TMF1*/*CCDC102B*/*CCDC102A, TMC2-8, COL5A1*/*COL11A1*/ *COL2A1*/*COL1A1*/*COL5A3*/*COL22A1*/*COL5A2*/*COL3A1*/*COL1A2*/*COL27A1*/*COL4A5*/*COL24A1*/*COL4A3*/ *COL4A1*/*COL4A6*/*COL7A1*/*COL4A4*/*COL16A1*/*COL4A2*/*COL18A1*/*COL9A1*/*COL17A1*/*COL15A1*/*COL9A3* /*COL28A1*/*COL9A2*/*COL13A1*/*COL6A1*/*COL25A1*/*COL21A1*/*COL6A2*/*COL2DA1*/*COL23A1*/*COLQ*/*EMID1* /*COL26A1*/*EDA, KCNG11KCNS31KCNC21KCNF11KCNA41KCNG41KCNA21KCNA61KCNA31KCND31 KCNG3*/*KCNA10*/*KCNA7*/*KCNA1*/*KCNS2*/*KCNV2*/*KCNS1, SLC26A3*/*SLC26A6*/*SLC26A5*/*SLC26A9*/ *SLC26A2*/*SLC26A1*/*SLC26A7*/*SLC26A8*/*SLC26A1, or VIT*/*COL12A1*/*COL6A3*/*COL6A6*/*COL14A1*/*COL6A5*/ *MATN1*/*MATN4*/*MATN2*/*VWA2*/*MATN3*/*VWA1.*

In accordance with a further related more preferred embodiment of the first aspect of the invention the disease is selected from muscular dystrophy (preferably Duchenne muscular dystrophy, caused by mutations in the *DMD gene*)*,* a laminopathy (caused by mutations in the *LMNA* gene), Marfan Syndrome (caused by mutations in the *FBN1* gene), cystic fibrosis (caused by mutations in the *CFTR* gene), retinitis pigmentosa (caused by mutations in the *RHO* gene), hypertrophic cardiomyopathy (caused by mutations in the *MYH7* gene), Li-Fraumeni Syndrome (caused by mutations in the *TP53* gene), Familial Hypercholesterolemia (caused by mutations in the *LDLR* gene), Hereditary Hemochromatosis (caused by mutations in the *HFE* gene), Achondroplasia (caused by mutations in the *FGFR3* gene), Tay-Sachs Disease (caused by mutations in the *HEXA* gene), Phenylketonuria (caused by mutations in the *PAH* gene), Congenital Contractural Arachnodactyly (caused by mutations in the *FBN2* gene), Intellectual disability/ developmental delay (caused by mutations in the *HNRNPH1* gene), Amyotrophic lateral sclerosis (caused by mutations in *SOD1* or *SPTLC1*)*,* neurodevelopmental deficits and epilepsy (caused by mutations in *ACTL6B*)*,* and hearing loss genetic diseases (caused by *GJB2, MYO7A, TMC1, COL11A2, KCNQ4, SLC2A64, or COCH).*

The above non-limiting but preferred examples of (i) genes with a missense, nonsense or in-frame indel mutation, (ii) the functional or sequence paralogs (also referred to herein as "compensating gene"), and (iii) diseases are interrelated. This interrelation can be taken from the following Table 1.

**Table 1**

| **Gene** | **Compensating gene** | **Disease** |
|---|---|---|
| *DMD* | *UTRN* | Duchenne muscular dystrophy |
| *FBN1* | *FBN2* | Marfan syndrome |
| *CFTR* | *TMEM16A* | Cystic fibrosis |
| *LMNA* | *NEFL* | Laminopathies |
| *RHO* | *OPN1MW, OPN1MW2, OPN1MW3,* | Retinitis pigmentosa |
| | *OPN1LW, OPN1SW, OPN3, OPN4, RRH, OPN5* | |
| *MYH7* | *MYH6* | Hypertrophic cardiomyopathy |
| *TP53* | *TP73, TP63* | Li-Fraumeni syndrome |
| *LDLR* | *LPR8, VLDLR, LRP1, LRP1B, LRP4, LRP2, EGF, NID1, NID2, LRP5, LRP6, LRP12, LRP10, LRP3* | Familial Hypercholesterolemia |
| *HFE* | *HLA-F, HLA-C, HLA-G, HLA-A, HLA-E, AZGP1, MR1, MICB, FCGRT, MICA, CD1A, CD1C, CD1D, CD18, CD1E, RAET1L, ULBP1, RAET1G, ULBP2, ULBP3, RAET1E* | Hereditary Hemochromatosis |
| *FGFR3* | *FGFR2, FGFR1, FGFR4, RET, KDR, FLT4, FLT1, KIT, FLT3, PDGFRB, PDGFRA, CSF1R, ROR2, TIE2, TEK, ROS1, MSTLR, ROR1, EPHA4, INSR, IGF1R, EPHA2, ALK, AXL, NTRK2, EPHA5, EPHA7, EPHA8, MET, ERBB4, EPHA3, EPHB3, TYRO3, MUSK, EPHB4, ERBB2, EPAH1, NTRK3, EPHB2, EGFR, EPHB1, EPHA6, MERTK, NRTK1, EPHA10, LTK, EPHB6, INSRR, ERBB3, DDR2, DDR1, LMTK2, RYK* | Achondroplasia |
| *HEXA* | *HEXB* | Tay-Sachs Disease |
| *PAH* | *TPH2, TPH1, TH* | Phenylketonuria |
| *FBN2* | *FBN1* | Congenital Contractural Arachnodactyly |
| *HNRNPH1* | *HRNPH2* | Intellectual disability/ developmental delay |
| *SOD1* | *CCS, SOD3* | Amyotrophic lateral sclerosis |
| *SPLTC1* | *SPTLC2, SPTLC3, ALAS1, GCAT, ALAS2* | Amyotrophic lateral sclerosis |
| *ACTL6B* | *ACTL6A, ACTB, ACTG1, ACTC1, ACTA2, ACTG2, ACTA1, ACTBL2, ACTR1A, ACTR1B, ACTL7B, ACTR3B, ACTRT2, ACTR3, ACTRT1, ACTL9, ACTRT3, ACTR2, ACTL7A, ACTL8, ACTR8, ACTR5, ACTR10, ACTR6, ACTL10, ACTR3C* | Neurodevelopmental deficits and epilepsy |
| *GJB2* | *GJB6, GJB1, GJB3, GJA8, GJB4, GJA3, GJA4, GJB5, GJA1, GJB7, GJA10, GJA9, GJA5, GJD2, GJC2, GJC1, GJD3, GJD4, GJET1* | Non-syndromic autosomal recessive deafness |
| *MYO7A* | *MYO7B, MYH6, MYH7, MYH8, MYH4, MYH1,MYH2, MYH3, MYH7B, MYH9, MYH13, MYH10, MYH15, MYO10, MYH14, MYO5B, MYO5A, MYO9B, MYO9A, MYO15A, MYOSC, MYO3A, MYO16, MYO18, MYO18A, MYO18B, MYO1E, MYO1C, MYO1A, MYO6, MYO3B, MYO1D, MYO1F, MYO1G, MYO1H, MYO19, CCDC158, CGNL1, TMF1, CCDC102B, CCDC102A* | Usher Syndrome Type 1B and non-syndromic hearing loss |
| *TMC1* | *TMC2-8* | Autosomal recessive non-syndromic hearing loss |
| *COL11A2* | *COLSA1, COL11A1, COL2A1, COL1A1, COL5A3, COL22A1, COL5A2, COL3A1, COL1A2, COL27A1, COL4A5, COL24A1, COL4A3, COL4A1, COL4A6, COL7A1, COL4A4, COL16A1, COL4A2, COL18A1, COL9A1, COL17A1, COL15A1, COL9A3, COL28A1, COL9A2, COL13A1, COL6A1, COL25A1, COL21A1, COL6A2, COL20A1, COL23A1, COLO, EMID1, COL26A1, EDA* | Stickler Syndrome and non-syndromic hearing loss |
| *KCNQ4* | *KCNG1, KCNS3, KCNC2, KCNF1, KCNA4, KCNG4, KCNA2, KCNA6, KCNA3, KCND3, KCNG3, KCNA10, KCNA7, KCNA1, KCNS2 KCNV2, KCNS1* | Autosomal dominant non-syndromic hearing loss |
| *SLC26A4* | *SLC26A3, SLC26A6, SLC26A5, SLC26A9, SLC26A2, SLC26A1, SLC26A7, SLC26A8, SLC26A11* | Pendred Syndrome |
| *COCH* | *VIT, COL12A1, COL6A3, COL6A6, COL14A1, COL6A5, MATN1, MATN4, MATN2, VWA2, MATN3, VWA1* | Autosomal dominant non-syndromic hearing loss |

The hearing loss genetic diseases are preferably selected from syndromic and/or non-syndromic autosomal (recessive or dominant) deafness, autosomal recessive non-syndromic hearing loss, Stickler Syndrome and non-syndromic hearing loss, autosomal dominant non-syndromic hearing loss, Pendred Syndrome and autosomal dominant non-syndromic hearing loss.

The above table also shows that paralogs can be genes that are separated by a number of gene duplication events. For instance, the *COCH* has all of *VIT, COL12A1, COL6A3, COL6A6, COL14A1, COL6A5, MATN1, MATN4, MATN2, VWA2, MATN3, VWA1* as paralogs.

In accordance with a preferred embodiment of the first aspect of the invention (1) the gene is associated with a disease in humans or is useful for treating a disease in humans and (2) the disease can be treated by the expression of the gene itself.

In case the gene is associated with a disease in humans a mutation in the gene is generally (at least in part) causative for the disease. In case the gene is useful for treating a disease in humans the gene is not necessarily mutated, but a subject having the disease benefits from the expression of the gene, for example, because the gene can cure or prevent /slow down the progression of the disease. In both cases such disease can be treated by the expression of the gene itself.

In accordance with a more preferred embodiment of the first aspect of the invention
the disease is myocardial infarction and the gene is VEGFA,
the disease is a tumor and the gene is FLT1,
the disease is sickle cell anemia and the gene is a globin gene, preferably HBG1/2,
the disease is thalassemia and the gene is HBB,
the disease is chronic anemia and the gene is EPO,
the disease is an autoimmune disease and the gene is an interleukin, preferably IL-10,
the disease is bone fractures or osteoporosis and the gene is BMP-2,
the disease is an ischemic disease and the gene is HIF1A or VEGFA,
the disease is hearing loss and the gene is OTOF,
the disease is vascular deficiency and the gene is NOS3,
the disease is due to oxidative stress and aging and the gene is FOXO3,
the disease is a metabolic disorder and the gene is PGC1alpha,
the disease is a metabolic disorder (e.g., type 2 diabetes, obesity) and the gene is GLP-1,
the disease is a cystic fibrosis and the gene is CFTR,
the disease is neuronal deficiency and senescence and the gene is SIRT1,
the disease is neuronal loss after stroke and the gene is NEUROD1,
the disease is neurodegeneration (e.g., in Parkinson's) and the gene is PRKN,
the disease is tumor growth and proliferation and the gene is PTEN,
the disease is cancer metastasis (metastatic cancer) and the gene is TIMP2,
the disease is blindness and the gene is CEP290 or RPE65, or
the disease age related macular degeneration (AMD) and the gene is FLT1.

The above preferred embodiment provides non-limiting but preferred examples of the genes being associated with a disease in humans or being useful for treating a disease in humans and the corresponding diseases that can be treated by the expression of these genes itself.

In accordance with a preferred embodiment of the first aspect of the invention the nucleic acid sequence encoding self-cleavable ribozyme comprises insulator sequences at the 5'- and 3'-ends that separate the nucleic acid sequence encoding the self-cleavable ribozyme from the sequences of the part of a gene according to (i), wherein the insulator sequences preferably comprise 8 to 16 base pairs, more preferably 10 to 14 and most preferably 12 base pairs and /orwherein the insulator sequences preferably comprise or consist of SEQ ID NO: 1 or a sequence being at least 80%, preferably at least 90% identical thereto.

The insulator sequences are optionally included and serve as buffers between the part of the genes and therein inserted the nucleic acid sequence encoding self-cleavable ribozyme. The insulator sequences facilitate the folding of the therapeutic nucleic acid molecule into the 3D conformation, wherein the self-cleavable ribozyme can cut the therapeutic nucleic acid molecule.

In accordance with the present invention, the term "percent (%) sequence identity" describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment, for two or more sequences or subsequences the percentage of amino acid residues or nucleotides that are the same (e.g. 70%, 75%, 80%, 85%, 90% or 95% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned.

Nucleotide and amino acid sequence analysis and alignment in connection with the present invention are preferably carried out using the NCBI BLAST algorithm (Stephen F. Altschul, Thomas L. Madden, Alejandro A. Schäffer, Jinghui Zhang, Zheng Zhang, Webb Miller, and David J. Lipman (1997), Nucleic Acids Res. 25:3389-3402). BLAST can be used for nucleotide sequences (nucleotide BLAST) and amino acid sequences (protein BLAST). The skilled person is aware of additional suitable programs to align nucleic acid sequences.

As defined herein, sequence identities of at least 70% identical, preferably at least 80% identical, more preferably at least 90% identical, and most preferred at least 95% are envisaged by the invention. However, also envisaged by the invention are with increasing preference sequence identities of at least 97.5%, at least 98.5%, at least 99%, at least 99.5%, at least 99.8%, and 100%.

In accordance with another preferred embodiment of the first aspect of the invention the therapeutic nucleic acid molecule further comprises
(a) a Kozak sequence comprising a start codon at the 5'-end of the part of a gene according to (i), wherein the Kozak sequence preferably comprises or consists of SEQ ID NO: 2 or a sequence being at least 80%, preferably at least 90% identical thereto, and/or
(b) a poly-A signal comprising a stop codon at the 3'-end of the part of a gene according to (i), wherein the poly-A signal preferably comprises or consists of SEQ ID NO: 3 (ATAAAA) or a sequence being at least 80%, preferably at least 90% identical thereto.

The Kozak consensus sequence (Kozak consensus or Kozak sequence) is a nucleic acid motif that functions as the protein translation initiation site in most eukaryotic mRNA transcripts.

The poly-A signal is the sequence motif being recognised by the RNA cleavage complex. It varies between groups of eukaryotes, but the most human polyadenylation sites contain the AAUAAA sequence, but this sequence is less common in plants and fungi.

In accordance with a further preferred embodiment of the first aspect of the invention the part of a gene according to (i) comprises or consists of exons E29 to E34 of the *DMD* gene and comprises or consists of preferably SEQ ID NO: 4 or a sequence being at least 80%, preferably at least 90% identical thereto.

The part of a gene according to (i) on the basis of which the invention is illustrated in the examples comprises or consists of exons E29 to E34 of the DMD gene. It preferably comprises or consists of preferably SEQ ID NO: 4 the part of a gene according to (i).

Altogether the exemplified "DMD RIBOZYME MINIGENE SEQUENCE" comprises or consists of the following SEQ ID NO: 5 
GCCACCATGGATTCACTTGAAAATTTGATGCGACATTCAGAGGATAACCCAAATCAGATTCGCATATTGGCACA
GACCCTAACAGATGGCGGAGTCATGGATGAGCTAATCAATGAGGAACTTGAGACATTTAATTCTCGTTGGAGG
 
GAACTACATGAAGAGGCTGTAAGGAGGCAAAAGTTGCTTGAACAGAGCATCCAGTCTGCCCAGGAGACTGAA
AAATCCTTACACTTAATCCAGGAGTCCCTCACATTCATTGACAAGCAGTTGGCAGCTTATATTGCAGACAAGGT
GGACGCAGCTCAAATGCCTCAGGAAGCCCAGAAAATCCAATCTGATTTGACAAGTCATGAGATCAGTTTAGAA
GAAATGAAGAAACATAATCAGGGGAAGGAGGCTGCCCAAAGAGTCCTGTCTCAGATTGATGTTGCACAGGTA
TATGTTATTTCAGAAACTAAGGAACGTGTTTTCGTTGGgcattatactccagtctatattCAAACAAACAAAGCGCGTCC
TGGATTCCACTTCGGGTACATCCAGCTGACGAGTCCCAAATAGGACGAAACGCGCCAAACAAACAAAggacaac
ttgattattggcAAGATTGGATTTGAGGACATTATTTGAAAACTATAATTTGATGTCTAAACAATGAGTGTTTTGCTC
TCTACATTTTAAAGATAAAATAGGTTAATAAATTACCTTTTATTTAGTATAACATTAAGAAAAAACTACTGAATC
ATTCTCCCAAATGTATGTTTTCCTGTGTTGGATGAATGGAAAAATACTGCTTTATTTTGTTTTTATTTTTCTGTAT
ATCTTCAGAAATAAAGGCAGATCTATCAATAAAAATAGGACCAGTTATTGTTTGAAAGGCAAAATTAAATCAGT
GCCTTTTTACACTGTCCTTACAGAAAAAATTACAAGATGTCTCCATGAAGTTTCGATTATTCCAGAAACCAGCCA
ATTTTGAGCAGCGTCTACAAGAAAGTAAGATGATTTTAGATGAAGTGAAGATGCACTTGCCTGCATTGGAAAC
AAAGAGTGTGGAACAGGAAGTAGTACAGTCACAGCTAAATCATTGTGTGAACTTGTATAAAAGTCTGAGTGAA
GTGAAGTCTGAAGTGGAAATGGTGATAAAGACTGGACGTCAGATTGTACAGAAAAAGCAGACGGAAAATCCC
AAAGAACTTGATGAAAGAGTAACAGCTTTGAAATTGCATTATAATGAGCTGGGAGCAAAGGTAACAGAAAGA
AAGCAACAGTTGGAGAAATGCTTGAAATTGTCCCGTAAGATGCGAAAGGAAATGAATGTCTTGACAGAATGG
CTGGCAGCTACAGATATGGAATTGACAAAGAGATCAGCAGTTGAAGGAATGCCTAGTAATTTGGATTCTGAAG
TTGCCTGGGGAAAGCCAAGCTTGGGGATCAATTCTCTAGAGCTCGCTGATCAGCCTCGACTGTGCCTTC
TAGTTGCCAGCCATCTGTTGTTTGCCCCTCCCCCGTGCCTTCCTTGACCCTGGAAGGTGCCACTCCCAC
TGTCCTTTCCTAATAAAATGAGGAAATTGCATCGCATTGTCTGAGTAGGTGTCATTCTATTCTGGGGG
GTGGGGTGGGGCAGGACAGCAAGGGGGAGGATTGGGAAGACAATAGCAGGCATGCTGGGGATGC
GGTGGGCTCTATGG
 or a sequence being at least 80%, preferably at least 90% identical thereto.

### Kozak sequence

*DMD exons (from E29 to E34, **start of exon marked with bold letter)***
Intron
Insulator
HHR-T3H38 active

bGH poly(A) signal
Stop codon
polyA

In accordance with a preferred embodiment of the first aspect of the invention the self-cleavable ribozyme is selected from Glucosamine-6-phosphate synthase (glmS) ribozymes, Hepatitis delta virus (HDV)-like ribozymes, hatchet ribozymes, hammerhead ribozymes, hairpin ribozymes, pistol ribozymes, twister ribozymes, twister sister ribozymes, *Neurospora* Varkud satellite (VS) ribozymes and hovlinc ribozymes, is preferably a hammerhead ribozyme (HHR) and is most preferably HHR-T3H38.

Various self-cleaving ribozymes that appear in nature catalyze the sequence-specific intramolecular cleavage of RNA and can be engineered to catalyze cleavage of appropriate substrates in an intermolecular fashion, thus acting as true catalysts. Non-limiting but preferred classes / families of suc self-cleaving ribozymes are Glucosamine-6-phosphate synthase (glmS) ribozymes, Hepatitis delta virus (HDV)-like ribozymes, hatchet ribozymes, hammerhead ribozymes, hairpin ribozymes, pistol ribozymes, twister ribozymes, twister sister ribozymes, Neurospora Varkud satellite (VS) ribozymes and hovlinc ribozymes (for review Peng eta al. (2021), RSC Chem. Biol., 2:1370-1383).

In a hammerhead ribozyme (HHR), particularly HHR-T3H38. The hammerhead ribozymes are a widespread class of self-cleaving RNAs that were originally found in plant viroids and satellites, catalyzing specific cleavage reactions during processing of rolling-circle transcripts. The cis-acting hammerhead ribozyme consists of a universally conserved junction sequence and three helices. The self-cleaving hammerhead ribozyme T3H38 is used in the appended examples and is known from Zhong et al. (2020), Nature Biotechnology, 38:169-175.

In accordance with a preferred embodiment of the first aspect of the invention the nucleic acid sequence encoding a self-cleavable ribozyme comprises or consists of SEQ ID NO: 6 or a sequence being at least 80%, preferably at least 90% identical thereto.

SEQ ID NO: 6 is the nucleic acid sequence encoding the self-cleaving hammerhead ribozyme T3H38.

In accordance with a preferred embodiment of the first aspect of the invention the gene being associated with a disease in humans comprises a mutation and the mutation is preferably a deletion, duplication, insertions, point mutation or inversion.

As discussed above disease (e.g. genetic diseases) can be caused by or associated with mutation in a gene. This mutation is preferably a deletion, duplication, insertions, point mutation or inversion.

In accordance with a preferred embodiment of the first aspect of the invention the therapeutic nucleic acid molecule comprises 6000 or less base pairs, preferably 5230 or less base pairs.

This size is preferred because AAV is capable of packaging and protecting recombinant genomes as large as 6.0 kb but not larger. AAV preferably packs no more than 5.23 kb.

The present application relates in a second aspect to a protein being encoded by open reading frame of the therapeutic nucleic acid molecule of the first aspect of the invention.

The definitions and preferred embodiment of the first aspect of the invention apply *mutatis mutandis* to the second aspect of the invention as far as being amenable therewith.

The term "protein" as used herein interchangeably with the term "polypeptide" describes linear molecular chains of amino acids, including single chain proteins or their fragments, containing at least 50 amino acids. The term "peptide" as used herein describes a group of molecules consisting of up to 49 amino acids, whereas the term "polypeptide" (also referred to as "protein") as used herein describes a group of molecules consisting of at least 50 amino acids. The term "peptide" as used herein describes a group of molecules consisting with increased preference of at least 15 amino acids, at least 20 amino acids at least 25 amino acids, and at least 40 amino acids. The group of peptides and polypeptides are referred to together by using the term "(poly)peptide". (Poly)peptides may further form oligomers consisting of at least two identical or different molecules. Furthermore, peptidomimetics of such proteins/(poly)peptides where amino acid(s) and/or peptide bond(s) have been replaced by functional analogues are also encompassed by the invention. Such functional analogues include all known amino acids other than the 20 gene-encoded amino acids, such as selenocysteine. The terms "(poly)peptide" and "protein" also refer to naturally modified (poly)peptides and proteins where the modification is effected e.g. by glycosylation, acetylation, phosphorylation and similar modifications which are well known in the art.

The present application relates in a third aspect to an expression vector, preferably an adeno-associated vector (AAV) comprising the therapeutic nucleic acid molecule of the first aspect of the invention.

The definitions and preferred embodiment of the first and second aspect of the invention apply *mutatis mutandis* to the third aspect of the invention as far as being amenable therewith.

The term "expression vector" in accordance with the invention means preferably a plasmid, cosmid, virus, bacteriophage or another vector used e.g. conventionally in genetic engineering which carries the nucleic acid molecule of the invention in expressible form. The nucleic acid molecule of the invention may, for example, be inserted into several commercially available vectors. Non-limiting examples include prokaryotic plasmid vectors, such as of the pUC-series, pBluescript (Stratagene), the pET-series of expression vectors (Novagen) or pCRTOPO (Invitrogen) and vectors compatible with an expression in mammalian cells like pREP (Invitrogen), pcDNA3 (Invitrogen), pCEP4 (Invitrogen), pMClneo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLX!N, pSIR (Clontech), pIRES-EGFP (Clontech), pEAK-10 (Edge Biosystems) pTriEx-Hygro (Novagen) and pCINeo (Promega). Examples for plasmid vectors suitable for Pichia pastoris comprise e.g. the plasmids pAO815, pPIC9K and pPIC3.5K (all Invitrogen).

The nucleic acid molecules inserted into the expression vector can e.g. be synthesized by standard methods. Ligation of the coding sequences to transcriptional regulatory elements and/or to other amino acid encoding sequences can also be carried out using established methods. Transcriptional regulatory elements (parts of an expression cassette) ensuring expression in prokaryotes or eukaryotic cells are well known to those skilled in the art. These elements comprise regulatory sequences ensuring the initiation of transcription (e. g., translation initiation codon, promoters, such as naturally-associated or heterologous promoters and/or insulators; see above), internal ribosomal entry sites (IRES) (Owens, Proc. Natl. Acad. Sci. USA 98 (2001), 1471-1476) and optionally poly-A signals ensuring termination of transcription and stabilization of the transcript. Additional regulatory elements may include transcriptional as well as translational enhancers. Preferably, the polynucleotide encoding the polypeptide/protein or fusion protein of the invention is operatively linked to such expression control sequences allowing expression in prokaryotes or eukaryotic cells. The vector may further comprise nucleic acid sequences encoding secretion signals as further regulatory elements. Such sequences are well known to the person skilled in the art. Furthermore, depending on the expression system used, leader sequences capable of directing the expressed polypeptide to a cellular compartment may be added to the coding sequence of the polynucleotide of the invention. Such leader sequences are well known in the art.

Furthermore, it is preferred that the vector comprises a selectable marker. Examples of selectable markers include genes encoding resistance to neomycin, ampicillin, hygromycine, and kanamycin. Specifically-designed vectors allow the shuttling of DNA between different hosts, such as bacteria-fungal cells or bacteria-animal cells (e. g. the Gateway system available at Invitrogen). An expression vector according to this invention is capable of directing the replication, and the expression, of the nucleic acid molecule and encoded protein of this invention.

The expression vector is preferably an adeno-associated vector (AAV) because AAV vectors are particularly suitable for gene therapy (for review see, for example, Naso et al. (2017), BioDrugs.; 31(4):317-33). AAV is a non-enveloped virus that can be engineered to deliver DNA to target cells, and has attracted a significant amount of attention in the field, especially in clinical-stage experimental therapeutic strategies. AAV has been shown to be safe and effective in preclinical and clinical settings. AAV can be used in a wide range of clinical applications in multiple diseases due its unique biological and biophysical properties. The AAV serotype is preferably selected from AAV1, AAV2, AAV4, AAV5, AAV6, AAV7, AAV8 and AAV9, noting that all these AAV serotypes were used in humans at least at the clinical trial stage and shows successful delivery of the transgene.

The present application relates in a fourth aspect to the therapeutic nucleic acid molecule of any one of claims of the first aspect or the protein of the second aspect or the expression vector of the third aspect for use in treating a disease that can be treated by the expression of the gene being associated with a disease in humans or being useful for treating a disease in humans or one of its functional or sequence paralog and preferably for treating a disease that is caused by (or associated with) a mutation in the gene from which the part according to the first aspect is derived.

The definitions and preferred embodiment of the first to third aspect of the invention apply *mutatis mutandis* to the fourth aspect of the invention as far as being amenable therewith.

By the provision of the therapeutic nucleic acid molecule, the protein and the expression vector of the invention the present invention provides a novel treatment option for treating a disease in a human.

The term "treating" comprises disease preventive treatment and curative treatment and is preferably a disease curative treatment. A curative treatment generally ameliorates or at least slows down the progression of one or more symptoms of the disease to be treated.

In accordance with a preferred embodiment of the fourth aspect the disease is selected from is selected from muscular dystrophy (preferably Duchenne muscular dystrophy, caused by mutations in the *DMD gene*)*,* a laminopathy (caused by mutations in the *LMNA* gene), Marfan Syndrome (caused by mutations in the *FBN1* gene), cystic fibrosis (caused by mutations in the *CFTR* gene), retinitis pigmentosa (caused by mutations in the *RHO* gene), hypertrophic cardiomyopathy (caused by mutations in the *MYH7 gene*)*,* Li-Fraumeni Syndrome (caused by mutations in the *TP53* gene), Familial Hypercholesterolemia (caused by mutations in the *LDLR* gene), Hereditary Hemochromatosis (caused by mutations in the *HFE* gene), Achondroplasia (caused by mutations in the *FGFR3* gene), Tay-Sachs Disease (caused by mutations in the *HEXA* gene), Phenylketonuria (caused by mutations in the *PAH* gene), Congenital Contractural Arachnodactyly (caused by mutations in the *FBN2* gene), Intellectual disability/ developmental delay (caused by mutations in the *HNRNPH1* gene), Amyotrophic lateral sclerosis (caused by mutations in *SOD1* or *SPTLC1*)*,* neurodevelopmental deficits and epilepsy (caused by mutations in *ACTL6B*)*,* and hearing loss genetic diseases (caused by *GJB2, MYO7A, TMC1, COL11A2, KCNQ4, SLC2A64, or COCH).*

Here and as also explained in connection with the first aspect above, a mutation in the gene is associated with a disease in humans, the gene has a functional or sequence paralog in the genome of humans, and the disease can be treated by one of its functional or sequence paralog(s) (wherein the expression of the functional or sequence paralog(s) is/are induced via TA).

Similarly, in accordance with another preferred embodiment of the fourth aspect the disease is selected from is selected from myocardial infarction (to be treated by the expression of VEGFA), a tumor (to be treated by the expression of FLT1), sickle cell anemia (to be treated by the expression of a globin gene, preferably HBB), chronic anemia (to be treated by the expression of EPO), an autoimmune diseases (to be treated by the expression of an interleukin, preferably IL-10), bone fractures or osteoporosis (to be treated by the expression of BMP-2), an ischemic disease (to be treated by the expression of HIF1A or VEGFA), hearing loss (to be treated by the expression of OTOF), blindness (to be treated by the expression of CEP290 or RPE65), or age related macular degeneration (AMD) (to be treated by the expression of FLT1).

Here and as also explained in connection with the first aspect above, the gene is associated with a disease in humans or is useful for treating a disease in humans and the disease can be treated by the expression of the gene itself (which expression is induced via TA).

In accordance with the present invention, the term "percent (%) sequence identity" describes the number of matches ("hits") of identical nucleotides/amino acids of two or more aligned nucleic acid or amino acid sequences as compared to the number of nucleotides or amino acid residues making up the overall length of the template nucleic acid or amino acid sequences. In other terms, using an alignment, for two or more sequences or subsequences the percentage of amino acid residues or nucleotides that are the same (e.g. 70%, 75%, 80%, 85%, 90% or 95% identity) may be determined, when the (sub)sequences are compared and aligned for maximum correspondence over a window of comparison, or over a designated region as measured using a sequence comparison algorithm as known in the art, or when manually aligned and visually inspected. This definition also applies to the complement of any sequence to be aligned.

Nucleotide and amino acid sequence analysis and alignment in connection with the present invention are preferably carried out using the NCBI BLAST algorithm (Stephen F. Altschul, Thomas L. Madden, Alejandro A. Schäffer, Jinghui Zhang, Zheng Zhang, Webb Miller, and David J. Lipman (1997), Nucleic Acids Res. 25:3389-3402). BLAST can be used for nucleotide sequences (nucleotide BLAST) and amino acid sequences (protein BLAST). The skilled person is aware of additional suitable programs to align nucleic acid sequences.

As defined herein, sequence identities at least 80% identical, preferably at least 90% identical, and most preferred at least 95% are envisaged by the invention. However, also envisaged by the invention are with increasing preference sequence identities of at least 97.5%, at least 98.5%, at least 99%, at least 99.5%, at least 99.8%, and 100%.

Regarding the embodiments characterized in this specification, in particular in the claims, it is intended that each embodiment mentioned in a dependent claim is combined with each embodiment of each claim (independent or dependent) said dependent claim depends from. For example, in case of an independent claim 1 reciting 3 alternatives A, B and C, a dependent claim 2 reciting 3 alternatives D, E and F and a claim 3 depending from claims 1 and 2 and reciting 3 alternatives G, H and I, it is to be understood that the specification unambiguously discloses embodiments corresponding to combinations A, D, G; A, D, H; A, D, I; A, E, G; A, E, H; A, E, I; A, F, G; A, F, H; A, F, I; B, D, G; B, D, H; B, D, I; B, E, G; B, E, H; B, E, I; B, F, G; B, F, H; B, F, I; C, D, G; C, D, H; C, D, I; C, E, G; C, E, H; C, E, I; C, F, G; C, F, H; C, F, I, unless specifically mentioned otherwise.

Similarly, and also in those cases where independent and/or dependent claims do not recite alternatives, it is understood that if dependent claims refer back to a plurality of preceding claims, any combination of subject-matter covered thereby is considered to be explicitly disclosed. For example, in case of an independent claim 1, a dependent claim 2 referring back to claim 1, and a dependent claim 3 referring back to both claims 2 and 1, it follows that the combination of the subject-matter of claims 3 and 1 is clearly and unambiguously disclosed as is the combination of the subject-matter of claims 3, 2 and 1. In case a further dependent claim 4 is present which refers to any one of claims 1 to 3, it follows that the combination of the subject-matter of claims 4 and 1, of claims 4, 2 and 1, of claims 4, 3 and 1, as well as of claims 4, 3, 2 and 1 is clearly and unambiguously disclosed.

The above considerations apply *mutatis mutandis* to all appended claims.

The figures show.

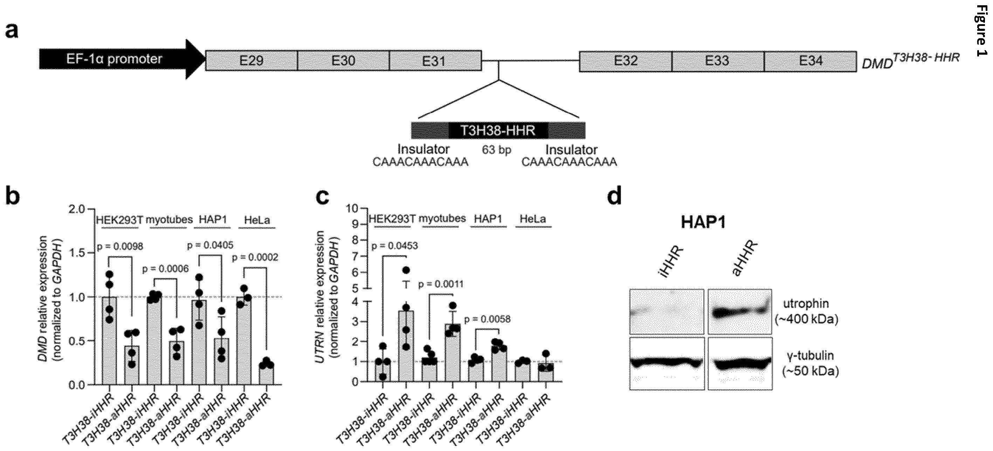

**Figure 1****. Utrophin upregulation induced by a self-cleaving ribozyme inserted into a *DMD^{WT}* minigene, a,** Schematic illustration of the *DMD* WT minigene containing a self-cleaving ribozyme (T3H38-HHR) inserted in intron 31, flanked by two insulator sequences. **(b-c)** Overexpression of the *DMD* WT minigene containing T3H38-HHR in WT cells results in *UTRN* upregulation. qPCR analysis of *DMD* **(b)** or *UTRN* **(c)** mRNA levels in WT HEK293T, myotubes, HAP1, and HeLa cells transfected with the *DMD* WT minigene with an inactive ribozyme (T3H38-iHHR) or an active ribozyme (T3H38-aHHR) (n = 4). Data are normalized to WT transfected with T3H38-iHHR and are mean ± s.d.; a two-tailed Student's t-test was used to calculate p values. **d**, Western blot showing increased utrophin protein levels in WT HAP1 cells transfected with T3H38-aHHR compared with cells transfected with T3H38-iHHR.

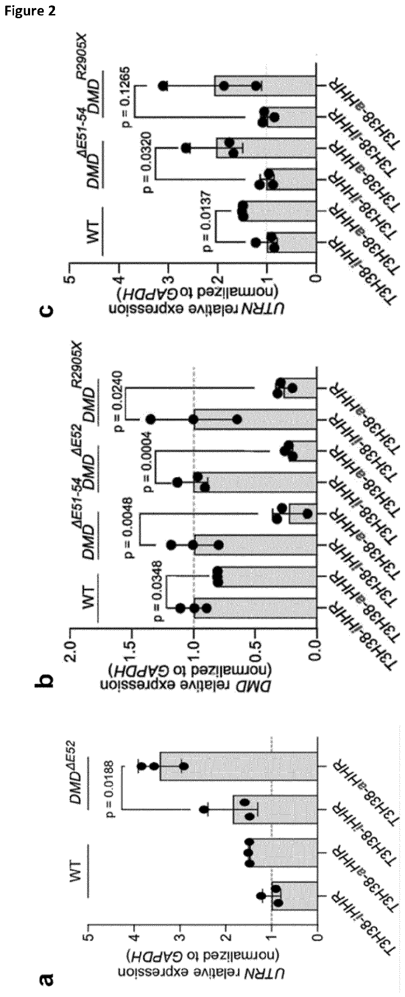

**Figure 2****. A *DMD^{WT}* minigene containing a self-cleaving ribozyme enhances *UTRN* upregulation in *DMD*^{*PTC*/*+*}myotubes. a,** qPCR analysis of *UTRN* mRNA levels in WT and *DMD^{ΔE52}* myotubes transfected with the *DMD^{T3H38-iHHR}* or *DMD^{T3H38-αHHR}* minigene (n = 3). **b,** qPCR analysis of exogenous *DMD* mRNA levels in WT, *DMD^{ΔE51-54}, DMD^{ΔE52},* and *DMD^{R2905X}* patient-derived myotubes transfected with the *DMD^{T3H38-iHHR}* or *DMD^{T3H38-αHHR}* ribozyme minigene (n = 3). **c,** qPCR analysis of *UTRN* mRNA levels in WT, *DMD^{ΔE51-54},* and *DMD^{R2905X}* patient-derived myotubes transfected with the *DMD^{T3H38-iHHR}* or *DMD^{T3H38-αHHR}* ribozyme minigene (n = 3). Data are normalized WT transfected with the *DMD^{T3H38-iHHR}* ribozyme minigene and are mean ± s.d.; a two-tailed Student's t-test was used to calculate p values.

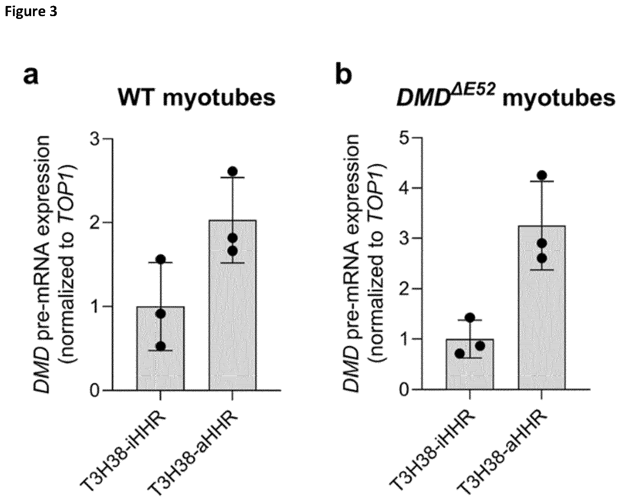

**Figure 3****. A *DMD^{WT}* minigene containing a self-cleaving ribozyme results in endogenous *DMD* pre-mRNA upregulation. a, b,** qPCR analysis of *DMD* pre-mRNA levels in WT **(a)** and *DMD^{ΔE52}* **(b)** myotubes transfected with the *DMD^{T3H38-iHHR}* or *DMD^{T3H38-αHHR}* minigene (n = 3).

The examples illustrate the claimed invention.

### Example 1- Ribozyme insertion into a minigene to induce transcriptional adaptation

Transcriptional adaptation (TA) is a cellular process in which the degradation of mutant mRNA modulates the expression of specific genes, known as adapting genes. It contributes to genetic robustness and is conserved across many species including zebrafish, *C*. *elegans,* and mice¹⁻⁶. At the interplay of mRNA surveillance mechanisms and small RNA-mediated gene regulation, TA operates independently of protein feedback loops, making it a unique mechanism to compensate for gene loss of function.

TA is triggered by premature termination codons (PTCs), which lead to mutant mRNA decay and translocation of the degradation fragments to the nucleus. We recently showed that other RNA destabilization events, such as CRISPR/Cas13-mediated and self-cleaving ribozyme-mediated mRNA cleavage could trigger TA independently of a PTC (Xie et al., in revision). Notably, ribozyme cleavage triggers the upregulation of adapting genes beyond the levels obtained with the other approaches.

We developed a TA-inducible approach where a ribozyme is inserted into a minigene (Fig. 1a), a minimal gene fragment. We found that TA could be enhanced by cleaving the mRNA transcribed from the minigene using a self-cleaving ribozyme instead of the insertion of a PTC. Self-cleaving ribozymes are short RNA molecules capable of catalyzing their own cleavage in a highly specific manner, which makes them a favorable tool to trigger TA reliably.

We have shown that TA also occurs in humans, in the context of Duchenne muscular dystrophy (DMD). The cleavage of a DMD/dystrophin transcript from a minigene by a ribozyme leads to the upregulation of *UTRN*/utrophin mRNA and protein levels in several different wild-type cell lines (Fig. 1b-d). We also observed increased *UTRN* mRNA levels in DMD patient-derived myotubes transfected with the active ribozyme compared with those transfected with the inactive ribozyme (Fig. 2a-c). It was previously shown that utrophin can functionally compensate for the lack of dystrophin⁷, and potentially improve prognosis in DMD patients. Thus, this method holds high promise for diseases where TA can be induced to activate compensatory genes. Notably, endogenous *DMD* pre-mRNA levels were also upregulated in wild-type as well as DMD patient-derived myotubes transfected with the active ribozyme compared with those transfected with the inactive ribozyme (Fig. 3a, b)

In summary, the disclosed method involves the insertion of a ribozyme sequence into a minigene, which is then introduced into target cells. Upon transcription, the ribozyme catalyzes the cleavage of the minigene and modulates the transcription of target genes through TA. This approach could activate a compensatory gene network in genetic diseases.

### References

1 Rossi, A. et al. Genetic compensation induced by deleterious mutations but not gene knockdowns. Nature 524, 230-233, doi:10.1038/nature14580 (2015).
2 El-Brolosy, M. et al. Genetic compensation is triggered by mutant mRNA degradation. bioRxiv (2018).
3 Ma, Z. et al. PTC-bearing mRNA elicits a genetic compensation response via Upf3a and COMPASS components. Nature 568, 259-263, doi:10.1038/s41586-019-1057-y (2019).
4 Jiang, Z. et al. Parental mutations influence wild-type offspring via transcriptional adaptation. Sci Adv 8, eabj2029, doi:10.1126/sciadv.abj2029 (2022).
5 Serobyan, V. et al. Transcriptional adaptation in Caenorhabditis elegans. eLife 9, doi:10.7554/eLife.50014 (2020).
6 Welker, J. M., Serobyan, V., Zaker Esfahani, E. & Stainier, D. Y. R. Partial sequence identity in a 25-nucleotide long element is sufficient for transcriptional adaptation in the Caenorhabditis elegans act-5/act-3 model. PLoS Genet 19, e1010806, doi:10.1371/journal.pgen.1010806 (2023).
7 Deconinck, A. E., Rafael, J. A., Skinner, J. A., Brown, S. C., Potter, A. C., Metzinger, L., Watt, D. J., Dickson, J. G., Tinsley, J. M., Davies, K. E. Utrophin-dystrophin-deficient mice as a model for Duchenne muscular dystrophy. Cell. 90, 717-27 (1997).
8 Zhong, G., Wang, H., He, W., Li, Y., Mou, H., Tickner, Z.J., Tran, M.H., Ou, T., Yin, Y., Diao, H., Farzan, M. A reversible RNA on-switch that controls gene expression of AAV-delivered therapeutics in vivo. Nat Biotechnol 38, 169-175 (2020).
9 Doherty, E.A, Doudna, J.A. Ribozyme structures and mechanisms. Annu Rev Biochem 69, 597-615 (2000).

## Claims

1. A therapeutic nucleic acid molecule comprising or consisting of an open reading frame comprising
(i) a part of a gene,
wherein the part comprises
(a) at least one intron and the flanking exons of the gene,
(b) at least two exons of the gene, wherein the first exon encodes the 5'UTR or the last exon encodes the 3'UTR,
(c) at least one exon of the gene preceded by an exogenous 5'UTR or followed by an exogenous 3'UTR, or
(d) at least one exon of the gene,
wherein the gene is associated with a disease in humans or is useful for treating a disease in humans,
wherein the disease can be treated by the expression of the gene itself or one of its functional or sequence paralogs; and
(ii) inserted within the part of a gene according to (i) a nucleic acid sequence encoding a self-cleavable ribozyme, wherein
(a') in (a) the nucleic acid sequence encoding a self-cleavable ribozyme is inserted into the intron,
(b') in (b) the nucleic acid sequence encoding a self-cleavable ribozyme is inserted into the 5' or 3'UTR,
(c') in (c) the nucleic acid sequence encoding a self-cleavable ribozyme is inserted into the exogenous 5' or 3'UTR, and
(d') in (d) the nucleic acid sequence encoding a self-cleavable ribozyme is inserted into at least one exon.

2. The therapeutic nucleic acid molecule of claim 1, wherein a mutation in the gene is associated with a disease in humans, wherein the gene has a functional or sequence paralog in the genome of humans, and wherein the disease can be treated by one of its functional or sequence paralog(s).

3. The therapeutic nucleic acid molecule of claim 2, wherein the gene is *DMD, LMNA, FBN1, CFTR, RHO, MYH7, TP53, LDLR, HFE, FGFR3, HEXA, PAH, FBN2, HNRNPH1, SOD1, SPTLC1, ACTL6B, GJB2, MYO7A, TMC1, COL11A2, KCNQ4, SLC26A4* or *COCH* and is preferably *DMD.*

4. The therapeutic nucleic acid molecule of claim 2 or 3, wherein the functional or sequence paralog is *UTRN, NEFL FBN2, TMEM16A, NEFL, OPN1MW*/*OPN1MW2*/*OPN1MW3*/*OPN1LW*/*OPN1SW*/*OPN3*/*OPN4*/*RRH*/*OPNS, MYH6, TP73*/*TP63, LPRS*/*VLDLR*/*LRP1*/*LRP18*/*LRP4*/*LRP2*/*EGF*/*NID1*/*NID2*/*LRPS*/*LRP6*/*LRP12*/*LRP10*/*LRP3, HLA-F*/*HLA-C*/*HLA-G*/*HLA-A*/*HLA-E*/*AZGP1*/*MR1*/*MICB*/*FCGRT*/*MICA*/*CD1A*/*CD1C*/*CD1D*/*CD18*/*CD1E*/*RAET1L*/*ULBP1*/*RAET1G*/*UL BP2*/*ULBP3*/*RAET1E, FGFR2*/*FGFR1*/*FGFR4*/*RET*/*KDR*/*FLT4*/*FLT1*/*KIT*/*FLT3*/*PDGFRB*/*PDGFRA*/*CSF1R*/*ROR2*/*TIE2*/*TEK*/ *ROS1*/*MST1R*/*ROR1*/*EPHA4*/*INSR*/*IGF1R*/*EPHA2*/*ALK*/*AXL*/*NTRK2*/*EPHAS*/*EPHA7*/*EPHAS*/*MET*/*E RBB4*/*EPHA3*/*EPHB3*/*TYR03*/*M USK*/*EPHB4*/*ERBB2*/*EPAH l*/*NTRK3*/*EPHB2*/*EGFR*/*EPHB1*/*EPHA6*/ *MERTK*/*NRTK1*/*EPHA10*/*LTK*/*EPH86*/*INSRR*/*ER883*/*DDR2*/*DDR1*/*LMTK2*/*RYK, HEXB, TPH2*/*TPH1*/*TH, FBN1, HRNPH2, CCS*/*SOD3, SPTLC2*/*SPTLC3*/*ALAS1*/*GCAT*/*ALAS2, ACTL6A*/*ACTB*/*ACTG1*/*ACTC1*/*ACTA2*/*ACTG2*/*ACTA1*/*ACTBL2*/*ACTR1A*/*ACTR1B*/*ACTL7B*/*ACTR3B* /*ACTRT2*/*ACTR3*/*ACTRTl*/*ACTL9*/*ACTRT3*/*ACTR2*/*ACTL7A*/*ACTL8*/*ACTR8*/*ACTR5*/*ACTR10*/*ACTR6* /*ACTL10*/*ACTR3C, GJ86*/*GJ81*/*GJ83*/*GJAS*/*GJ84*/*GJA3*/*GJA4*/*GJ85*/*GJA1*/*GJ87*/*GJA10*/*GJA9*/*GJAS*/*GJD2*/*GJC2*/*GJC1* /*GJD3*/*GJD4*/*GJET1, MYO7B*/*MYH6*/*MYH7*/*MYH8*/*MYH4*/*MYH1*/*MYH2*/*MYH3*/*MYH7B*/*MYH9*/*MYH13*/*MYH10*/*MYH 15*/*MYO10*/*MYH14*/*MYO58*/*MYO5A*/*MYO98*/*MYO9A*/*MYO15A*/*MYO5C*/*MYO3A*/*MYO16*/*MYO 1B*/*MYO18A*/*MYO18B*/*MYO1E*/*MYO1C*/*MYO1A*/*MYO6*/*MYO3B*/*MYO1D*/*MYO1F*/*MYO1G*/*MYO 1H*/*MYO19*/*CCDC158*/*CGNL1*/*TMF1*/*CCDC1028*/*CCDC102A, TMC2-8, COL5A1*/*COL11A1*/*COL2A1*/*COL1A1*/*COL5A3*/*COL22A1*/*COL5A2*/*COL3A1*/*COL1A2*/*COL27A1*/*CO L4A5*/*COL24A1*/*COL4A3*/*COL4A1*/*COL4A6*/*COL7A1*/*COL4A4*/*COL16A1*/*COL4A2*/*COL18A1*/*COL9 A1*/*COL17A1*/*COL15A1*/*COL9A3*/*COL28A1*/*COL9A2*/*COL13A1*/*COL6A1*/*COL25A1*/*COL21A1*/*COL 6A2*/*COL20A1*/*COL23A1*/*COLQ*/*EMID1*/*COL26A1*/*EDA, KCNG1*/*KCNS3*/*KCNC2*/*KCNF1*/*KCNA4*/*KCNG4*/*KCNA2*/*KCNA6*/*KCNA3*/*KCND3*/*KCNG3*/*KCNA10*/ *KCNA7*/*KCNA1*/*KCNS2*/*KCNV2*/*KCNS1, SLC26A3*/*SLC26A6*/*SLC26A5*/*SLC26A9*/*SLC26A2*/*SLC26A1*/*SLC26A7*/*SLC26A8*/*SLC26A1, or VIT*/*COL12A1*/*COL6A3*/*COL6A6*/*COL14A1*/*COL6A5*/*MATN1*/*MATN4*/*MATN2*/*VWA2*/*MATN3*/*V WA1.*

5. The therapeutic nucleic acid molecule of any one of claims 2 to 4, wherein the disease is selected from muscular dystrophy (preferably Duchenne muscular dystrophy, caused by mutations in the *DMD gene*)*,* a laminopathy (caused by mutations in the *LMNA* gene), Marfan Syndrome (caused by mutations in the *FBN1* gene), cystic fibrosis (caused by mutations in the *CFTR* gene), retinitis pigmentosa (caused by mutations in the *RHO* gene), hypertrophic cardiomyopathy (caused by mutations in the *MYH7* gene), Li-Fraumeni Syndrome (caused by mutations in the *TP53* gene), Familial Hypercholesterolemia (caused by mutations in the *LDLR* gene), Hereditary Hemochromatosis (caused by mutations in the *HFE* gene), Achondroplasia (caused by mutations in the *FGFR3* gene), Tay-Sachs Disease (caused by mutations in the *HEXA* gene), Phenylketonuria (caused by mutations in the *PAH* gene), Congenital Contractural Arachnodactyly (caused by mutations in the *FBN2* gene), Intellectual disability/ developmental delay (caused by mutations in the *HNRNPH1* gene), Amyotrophic lateral sclerosis (caused by mutations in *SOD1* or *SPTLC1*)*,* neurodevelopmental deficits and epilepsy (caused by mutations in *ACTL6B*)*,* and hearing loss genetic diseases (caused by *GJB2, MYO7A, TMC1, COL11A2, KCNQ4, SLC2A64, or COCH).*

6. The therapeutic nucleic acid molecule of claim 1, wherein the gene is associated with a disease in humans or is useful for treating a disease in humans and wherein the disease can be treated by the expression of the gene itself.

7. The therapeutic nucleic acid molecule of claim 6, wherein
the disease is myocardial infarction and the gene is VEGFA,
the disease is a tumor and the gene is FLT1,
the disease is sickle cell anemia and the gene is a globin gene, preferably HBG1/2,
the disease is thalassemia and the gene is HBB,
the disease is chronic anemia and the gene is EPO,
the disease is an autoimmune disease and the gene is an interleukin, preferably IL-10,
the disease is bone fractures or osteoporosis and the gene is BMP-2,
the disease is an ischemic disease and the gene is HIF1A or VEGFA,
the disease is hearing loss and the gene is OTOF,
the disease is vascular deficiency and the gene is NOS3,
the disease is due to oxidative stress and aging and the gene is FOXO3,
the disease is a metabolic disorder and the gene is PGC1alpha,
the disease is a metabolic disorder (e.g., type 2 diabetes, obesity) and the gene is GLP-1,
the disease is a cystic fibrosis and the gene is CFTR,
the disease is neuronal deficiency and senescence and the gene is SIRT1,
the disease is neuronal loss after stroke and the gene is NEUROD1,
the disease is neurodegeneration (e.g., in Parkinson's) and the gene is PRKN,
the disease is tumor growth and proliferation and the gene is PTEN,
the disease is cancer metastasis and the gene is TIMP2,
the disease is blindness and the gene is CEP290 or RPE65, or
the disease age related macular degeneration (AMD) and the gene is FLT1.

8. The therapeutic nucleic acid molecule of any one of claims 1 to 6, wherein the nucleic acid sequence encoding self-cleavable ribozyme comprises insulator sequences at the 5'- and 3'-ends that separate the nucleic acid sequence encoding the self-cleavable ribozyme from the sequences of the part of a gene according to (i), wherein the insulator sequences preferably comprise 8 to 16 base pairs, more preferably 10 to 14 and most preferably 12 base pairs and /orwherein the insulator sequences preferably comprise or consist of SEQ ID NO: 1 or a sequence being at least 80%, preferably at least 90% identical thereto.

9. The therapeutic nucleic acid molecule of any one of claims 1 to 8, wherein the therapeutic nucleic acid molecule further comprises
(a) a Kozak sequence comprising a start codon at the 5'-end of the part of a gene according to (i), wherein the Kozak sequence preferably comprises or consists of SEQ ID NO: 2 or a sequence being at least 80%, preferably at least 90% identical thereto, and/or
(b) a poly-A signal comprising a stop codon at the 3'-end of the part of a gene according to (i), wherein the poly-A signal preferably comprises or consists of SEQ ID NO: 3 or a sequence being at least 80%, preferably at least 90% identical thereto.

10. The therapeutic nucleic acid molecule of any one of claims 1 to 9, wherein the part of a gene according to (i) comprises or consists of exons E29 to E34 of the *DMD* gene and comprises or consists of preferably SEQ ID NO: 4 or a sequence being at least 80%, preferably at least 90% identical thereto.

11. The therapeutic nucleic acid molecule of any one of claims 1 to 10, wherein the self-cleavable ribozyme is selected from Glucosamine-6-phosphate synthase (glmS) ribozymes, Hepatitis delta virus (HDV)-like ribozymes, hatchet ribozymes, hammerhead ribozymes, hairpin ribozymes, pistol ribozymes, twister ribozymes, twister sister ribozymes, *Neurospora* Varkud satellite (VS) ribozymes and hovlinc ribozymes, is preferably a hammerhead ribozyme (HHR) and is most preferably HHR-T3H38.

12. The therapeutic nucleic acid molecule of any one of claims 1 to 11, wherein the nucleic acid sequence encoding a self-cleavable ribozyme comprises or consists of SEQ ID NO: 6 or a sequence being at least 80%, preferably at least 90% identical thereto.

13. A protein being encoded by open reading frame of the therapeutic nucleic acid molecule of any one of claims 1 to 12.

14. An expression vector, preferably an adenoassociated vector (AAV) comprising the therapeutic nucleic acid molecule of any one of claims 1 to 12.

15. The therapeutic nucleic acid molecule of any one of claims 1 to 12 or the protein of claim 13 or the expression vector of claim 14 for use in treating a disease that can be treated by the expression of the gene being associated with a disease in humans or being useful for treating a disease in humans or one of its functional or sequence paralogs and preferably for treating a disease that is caused by a mutation in the gene from which the part according to claim 2 (i) is derived.
